# EUROPEAN PATENT SPECIFICATION

(11) **EP 2 717 788 B1**
(45) Date of publication and mention of the grant of the patent: **10.08.2016**
(21) Application number: 12796665.3
(22) Date of filing: 11.06.2012
(51) Int. Cl.: A61B 17/08, A61B 17/11, A61B 17/00

(54) **BIO-ABSORBABLE MICRO-CLIP FOR MINIMAL ACCESS WOUND CLOSURE**
BIOABSORBIERBARER MICROCLIP FÜR MINIMALINVASIVEN WUNDVERSCHLUSS
MICRO-AGRAFE BIO-ABSORBABLE POUR UNE FERMETURE DE PLAIE À ACCÈS MINIMAL

(30) Priority: 09.06.2011 US 201161495035 P
(43) Date of publication of application: 16.04.2014
(73) Proprietor: Singapore Health Services Pte Ltd, Singapore 168753 (SG); National University of Singapore, Singapore 119077 (SG)
(72) Inventor: LAU, David Pang Cheng, Singapore 168753 (SG); CHUI, Chee Kong, Singapore 168753 (SG); CHNG, Chin Boon, Singapore 168753 (SG); CHOO, Jun Quan, Singapore 168753 (SG); YANG, Tao, Singapore 650298 (SG); TEOH, Swee Hin, Singapore 427305 (SG)
(74) Representative: Fyles, Julie Marie
(86) International application number: PCT/SG2012/000211
(87) International publication number: WO 2012/169974

(56) References cited:
- EP-A1- 0 852 128
- EP-A1- 1 787 604
- EP-A1- 2 127 606
- SU-A1- 1 456 109
- US-A- 4 217 902
- US-A- 4 217 902
- US-A- 4 505 274
- US-A- 5 049 153
- US-A- 5 154 189
- US-A- 5 236 440
- US-A- 5 618 311
- US-A1- 2002 111 641
- US-A1- 2004 050 393
- US-A1- 2008 173 693
- US-A1- 2008 319 455

## Description

### Technical Field

The present disclosure relates to a wound closure device, an applicator therefore, and method of using the same. More specifically, the present disclosure relates to bio-absorbable micro-clips for use in microsurgical applications.

### Background

The vocal folds, located within the larynx at the top of the trachea are necessary for phonation. The vocal fold (vocal cord) is a layered structure that includes a stratified squamous epithelium, superficial lamina propria, and deep connective tissue. Both the epithelium and lamina propria layers are involved in phonation. Injuries to the vocal fold may occur due to chronic overuse, trauma, lesions, polyps, nodules, cancer, surgical operations and the like.

Healing of the vocal folds often results in formation of scar tissue. Scaring of the vocal fold may result in deformation of the vocal fold edge and disruption of the viscosity and flexibility of the lamina propria. Scaring of the vocal fold may increase the effort required for phonation and breathing, as well as causing dysphonia and vocal fatigue.

The level of scaring of the vocal fold is impacted by many features, including, for example, the cause of the injury, the level of inflammation induced by the injury and subsequent remediary measures such as surgical intervention. When surgical intervention is required, the type of surgery, time required for completion of the surgery, manipulation of the vocal folds, instruments used in the vicinity thereof, and means of closing wounds (either surgical or trauma induced), all impact the resulting level of scar tissue on the vocal fold. Furthermore, reaction by the body's immune system and the healing process itself also contribute to scar tissue formation.

Advances in surgical procedures and technologies have provided for the replacement of major surgical events with minimal access surgery. Minimal access surgery is commonly used in procedures ranging from appendectomies to vascular repair. It provides for smaller incisions, quicker healing, and less exposure of internal organs.

Minimal access surgery is typically accomplished utilizing a trocar along with other microsurgical devices such as forceps, scopes, clamps, suction devices and the like. These devices are used to view, dissect, and correct defects within a patient through a small opening. Equally small wound closure devices such as staples, sutures, and the like have been developed for use in conjunction with microsurgical devices.

Due to their minimally invasive structure, sutures are often used in laryngeal surgery. However, limited instrument movement, reduced tactile feedback, and loss of stereopsis create challenges. Other wound closure devices, such as staples, crush the tissue injuring delicate mucosal tissue.

SU 1,456,109 represents the closest prior art and describes a fastener according to the preamble of claim 1.

Alternatives to wound closure devices, such as bio-adhesives are often used in situations where application of a wound closure device is more difficult due to structural or access limitations. However, bio-adhesives may increase scar tissue formation, seal too quickly or too slowly preventing proper positioning of the tissue, and have low tensile strength. These concerns are especially valid in minimal access surgeries that occur in areas with access and structural limitations but require high tensile strength and low scar tissue formation in order to prevent surgical complications and achieve the desired result.

Laryngeal surgery occurs in an area of the body with limitations caused by both access to the location and physiology. Wound closure methods for use in the larynx require a device with high tensile strength in order to allow for proper functioning of the vocal cords without reopening the wound. Wound closure methods and devices used in laryngeal surgery should penetrate the vocal fold only as deeply as necessary to ensure closure and not extend from the closure in such a manner as to damage the mucosal tissue or the opposing vocal fold. Such wound closure devices should absorb into the body in a timely fashion so as to minimize scaring. Any absorbable type wound closure device should require minimal time for application and not induce inflammation at the site of the wound so as to reduce scar tissue formation. The wound closure device should be more like a suture but easier to apply, providing minimal tissue penetration with maximal hold, pinching the tissue without damaging it.

Accordingly, there is a need for a wound closure device and method of application thereof for use in laryngeal surgery.

### Summary

The present disclosure describes, a bio-absorbable wound closure clip including an arcuate body; and at least two teeth carried by the arcuate body, wherein the bio-absorbable wound closure clip penetrates without crushing tissue.

The present invention is defined by the features of independent claim 1. Preferred embodiments are defined in the dependent claims.

The present disclosure further teaches, a method of closing a wound in minimal access surgery including applying one or more bio-absorbable wound closure clips to a wound during minimal access surgery, and closing the one or more bio-absorbable wound closure clips to ring-like shapes on said wound, wherein upon closing the bio-absorbable wound closure clips penetrate tissue without crushing.

The disclosure further teaches a plurality of bio-absorbable wound closure clips disposed in a substantially elliptical configuration relative to each other and securing a first portion of a tubular tissue structure to a second portion of a tubular tissue structure, each bio-absorbable wound closure clip within the plurality of bio-absorbable wound closure clips comprising an arcuate body and at least two teeth carried by the arcuate body, wherein said bio-absorbable wound closure clip penetrates without crushing tissue.

In another aspect, the disclosure comprises a kit comprising a number of bio-absorable wound closure clips as described above.

The disclosure further teaches a surgical instrument including a handle assembly; a shaft assembly; and a rotatable end assembly, wherein the rotatable end assembly comprises arms, which do not touch upon full closure.

The present disclosure further teaches a surgical instrument including forceps having cupped distal ends configured for holding an arcuate body of a bio-absorbable wound closure clip.

The present disclosure also teaches a kit for wound closure including forceps having cupped distal ends configured for holding an arcuate body of a bio-absorbable wound closure clip; and a number of bio-absorbable wound closure clips as described above.

In addition to the illustrative aspects, embodiments, and features will become apparent by reference to the drawings and the following detailed description.

### Brief Description of the Drawings

Embodiments of the disclosure are described herein with reference to the drawings in which:
**FIG. 1** is a depiction of an example of a bio-absorbable wound closure clip of the present disclosure;
**FIG. 2** is a depiction of an example of a bio-absorbable wound closure clip upon deformation of the wound closure device;
**FIG. 3** is a depiction of an example of a bio-absorbable wound closure clip in use in closing a vocal fold;
**FIG. 4** is a depiction of an example of a surgical instrument of the present disclosure;
**FIG. 5** is an expanded view of a portion of the surgical instrument of FIG. 4;
**FIG. 6** is an expanded view of a portion of the surgical instrument of FIG. 4;
**FIG. 7** is a depiction of the movement of an example of a surgical instrument of the
present disclosure;
**FIG. 8** is an embodiment of a bio-absorbable wound closure clip of the present invention;
**FIG. 9** is an alternate example of a bio-absorbable wound closure clip of the present disclosure;
**FIG. 10** is a graph of the degradation profile of examples of a bio-absorbable wound closure clip of Example 1;
**FIG. 11** is a graph of the tensile strength of an example of a bio-absorbable wound closure clip;
**FIG. 12** is a graph of the tensile strength of an example of a bio-absorbable wound closure clip of the present disclosure as compared to that of a suture; and
**FIG. 13** is a graph of the tensile strength of an example of a bio-absorbable wound closure clip of the present disclosure as compared to a suture.

### Detailed Description

In the following detailed description, reference is made to the accompanying drawings, which form a part hereof. In the drawings, similar symbols typically identify similar components, unless context dictates otherwise. The illustrative embodiments and examples described in the detailed description, drawings, and claims are not meant to be limiting.

The present disclosure provides a bio-absorbable wound closure clip suitable for use in minimal access surgery. Specifically the bio-absorbable wound closure clip can be used in areas of the body with limitations caused by access to a target bodily location and/or physiological structure. The bio-absorbable wound closure clip has a high tensile strength and induces little or minimal scar tissue formation *in-situ*. The bio-absorbable wound closure clip has a minimal penetration depth and absorbs into the body in a timely fashion so as to minimize scaring. The bio-absorbable wound closure clip does not crush target tissue (i.e., the clip at least substantially avoids crushing target tissue, such as mucosal tissue) and does not cause irritation to proximal, adjacent, or contralateral tissue. The bio-absorbable wound closure clips of the present disclosure require minimal time for application and avoid inducing excessive or substantial inflammation at a target bodily application site such as a the wound, thereby reducing or minimizing scar tissue formation.

The bio-absorbable wound closure clip is similar in penetration to a suture, providing minimal tissue penetration with maximal hold, pinching the tissue without damaging it. Unlike a suture, however, the bio-absorbable wound closure clip can be easily, reliably, and quickly applied. The bio-absorbable wound closure clip can be applied with a minimal amount of force thus reducing, minimizing, or preventing unnecessary or additional tissue damage. The bio-absorbable wound closure clip can be applied to mucosal tissue surfaces in situations in which tactile feedback is reduced making suture insertion difficult.

Although the present disclosure discusses primarily laryngeal and vocal fold surgery, the wound closure device and surgical instrument of the present disclosure can be used for essentially any type of minimal access surgery. Minimal access surgery can include microsurgery. Microsurgeries can include connecting bodily tissues, for example, muscles, tendons, and the like. Examples of microsurgeries that can be performed using the bio-absorbable wound closure clip of the present disclosure include surgical procedures performed in association with one or more of, without limitation, laryngeal surgery, vocal fold surgery, ocular surgery, nasal surgery, sinus surgery, endoscopic surgeries, closure of internal tubular tissue structures, skull base surgeries and the like, or any other surgical procedures.

The bio-absorbable wound closure clip can be formed from any deformable, inert, bio-absorbable material having a high tensile strength. For example, the bio-absorbable wound closure device can be formed from bio-absorbable metals, metal alloys, bio-absorbable metal-polymer composites, and/or one or more bio-absorbable polymers. Bio-absorbable metals that can be used include, for example, magnesium, calcium, strontium, and the like. Bio-absorbable metal alloys of, for example, magnesium, calcium, strontium, aluminum, zinc, manganese, silicon, yttrium, and combinations of these can also be used. Bio-absorbable polymers can be used to form the clip or as a coating on the bio-absorbable wound closure clip. Bio-absorbable polymers that can be used include polylactic acid, polyglycolic acid, polycaprolactone, poly dioxananone, poly trimethylene carbonate, and copolymers and blends thereof. In embodiments, the bio-absorbable wound closure clip can be formed from about 90% to about 99.9% magnesium. In some embodiments, the bio-absorbable wound closure clip can be formed from 99.5% magnesium.

In some embodiments, the material forming the bio-absorbable wound closure clip can be treated (e.g., surface treated). Treatment can include, for example, polishing, coating (e.g., bio-absorbable polymer coating, or gold coating), hydrogen embrittlement, stress corrosion, acid corrosion and the like and combinations of these. In some embodiments, the material forming the bio-absorbable wound closure clip can be surface abraded. In some embodiments, the material forming the bio-absorbable wound closure clip can be coated with a bio-absorbable polymer coating. In an embodiment, the material forming the bio-absorbable wound closure clip can be coated with poly-ε-caprolactone. In some embodiments, the bio-absorbable wound closure clip can be coated with gold. Treatment can be used to alter parameters of the bio-absorbable wound closure clip such as tensile strength and bio-degradation / resorption properties. For instance, in some embodiments, gold coating can accelerate bio-resorbability properties of the clip(s). Additionally, gold coating can further reduce or minimize tissue scarring.

In several embodiments, the bio-absorbable wound closure clip of the present disclosure has a thickness and structure suitable for efficient degradation and bio-absorption while maintaining tensile strength necessary for holding the wound closed during phonation. Additionally, the bio-absorbable wound closure clip can be stable enough to last for a target or intended time period such as about 2 weeks *in situ* so as to allow for healing to at least substantially occur. In embodiments, the bio-absorbable wound closure clip can have a thickness of about 0.15 mm to about 0.4 mm. In a number of embodiments, the bio-absorbable wound closure clip can have a thickness of about 0.35 mm to 0.2 mm. Alternate thicknesses can be used for various applications requiring longer or shorter duration *in situ* and/or greater or lesser tensile strength.

The epithelial layers of the vocal fold, including the lamina propria, have a depth of about 1 mm before the deep vocal ligaments and muscles are reached. The size of the bio-absorbable wound closure clip can be such that upon deformation, the bio-absorbable wound closure clip penetrates less than or equal to about 1 mm. Additionally, the bio-absorbable wound closure clip can be formed so that it does not extend from a wound in such a way as to damage or inflame the contra-lateral vocal fold or lower airway.

In embodiments, the bio-absorbable wound closure clip can have a major diameter from about 2 mm to about 4 mm. In embodiments, the major diameter of the bio-absorbable wound closure clip is about 3.5 mm. In embodiments, upon deformation, the bio-absorbable wound closure clip can have a diameter of from about 2.5 mm to about 3 mm. In embodiments, the bio-absorbable wound closure clip can have a diameter upon deformation of about 2 mm. Alternate diameters can be used depending on the location that the bio-absorbable wound closure clip is to be used.

The tensile strength of the bio-absorbable wound closure clip is dependent upon several factors, for example, thickness of the bio-absorbable wound closure clip, the materials used to construct the bio-absorbable wound closure clip, coating on the bio-absorbable wound closure clip, treatment applied to the bio-absorbable wound closure clip, and the like. In embodiments, the bio-absorbable wound closure clip can have a tensile strength from about 1500 mN to about 2500 mN. In embodiments, the bio-absorbable wound closure clip can have a tensile strength of about 1800 mN.

In general, application of the bio-absorbable wound closure clip in situ can take less than about 10 seconds, or less than about 5 seconds (e.g., from about 1 second to about 3 seconds). By contrast, suture application can take from about 15 to about 30 minutes. Thus application time for the bio-absorbable wound closure clip is considerably reduced even if multiple bio-absorbable wound closure clips are applied.

In embodiments, the bio-absorbable wound closure clip can act as a splint, wrapping around a vocal fold. The bio-absorbable wound closure clip apposes the epithelial edges and holds down the flap thereby synergistically providing advantageous properties of both sutures and glue. In embodiments, the bio-absorbable wound closure clip is not harmful upon inhalation and does not lodge in the airway or induce inflammation when inhaled.

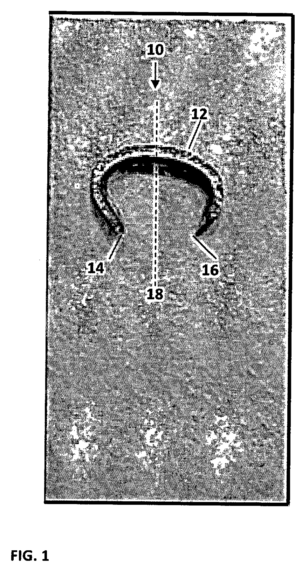

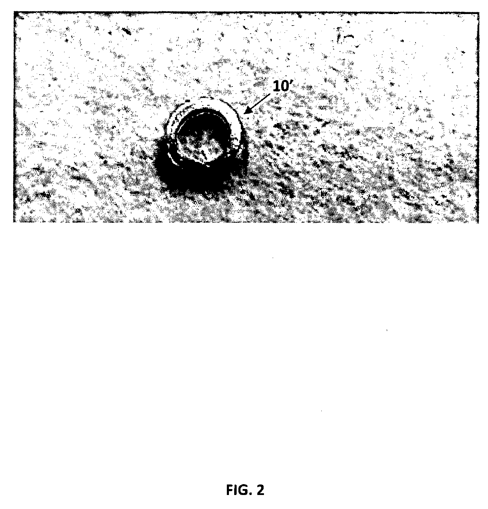

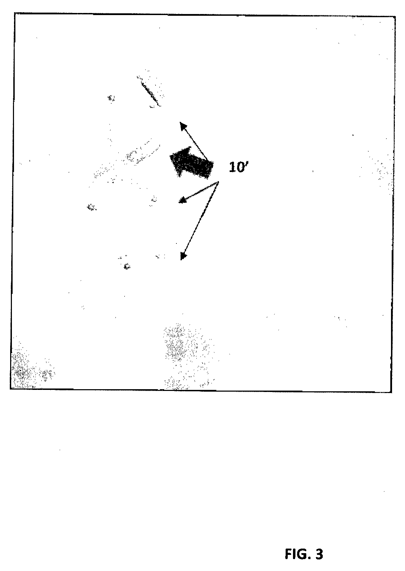

As depicted in **FIG. 1**, the bio-absorbable wound closure clip 10 can include an arcuate body 12 and teeth 14/16. As an example, the arcuate body can be arcuate at an angle of incidence of between about 20° and about 40° to the vertical 18. The arcuate body can be arcuate at an angle of incidence of about 30° to the vertical 18. Although described herein as arcuate, in other examples, the bio-absorbable wound closure clip 10 can be C-shaped, clip shaped, micro-clip shaped, and the like. The bio-absorbable wound closure clip 10 is further depicted in **FIG. 2** in a closed position (10'). Upon insertion into target tissue such as a wound, the bio-absorbable wound closure clip 10 is closed into an elliptical, circular, or "ring-like" shape 10'. The circular bio-absorbable wound closure clip 10' is depicted in **FIG. 3** as used in the closure of a vocal fold.

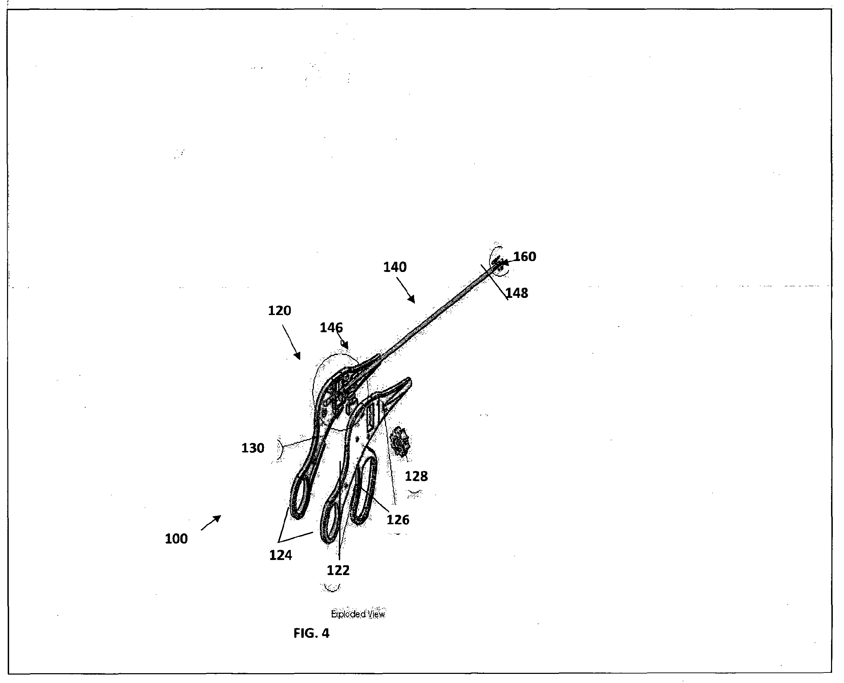

In some examples, the arcuate body of the bio-absorbable wound closure clip can lock, and hence can include one or more locking structures, mechanisms, or means configured for securing the bio-absorbable wound closure clip in a closed or locked position. Such locking structures or mechanisms are known in the art for locking clips. Application of a bio-absorbable wound closure clip to the vocal fold should be rapid and any surgical instrument used to apply the bio-absorbable wound closure clip should be adaptable and have a great degree of flexibility. A surgical instrument that can be used for application of the bio-absorbable wound closure clip of the present disclosure is depicted in **FIG. 4**. The surgical instrument 100 includes a handle assembly 120, a shaft assembly 140 connected at a proximal end 146 to said handle assembly 120; and, a rotatable end assembly 160 connected to said shaft assembly 140 at a distal end 148.

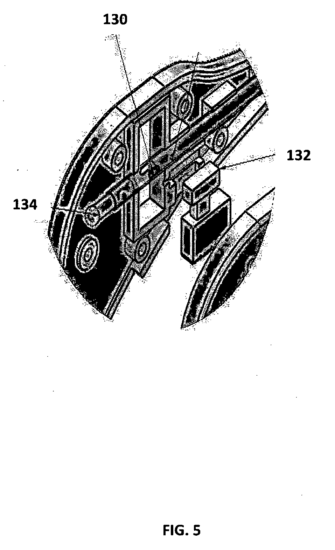

The handle assembly 120 includes exterior 122 and interior 130. Exterior 122 of handle assembly 120 includes a stationary handle 124, a grip handle 126, and knob 128. The interior 130 of the handle assembly 120 is further depicted in **FIG. 5**. Interior 130 if handle assembly 120 includes lock 132 and joint 134.

Shaft assembly 140 includes inner shaft 142 and outer shaft 144. Outer shaft 144 is encased at a proximal end 146 within interior 130 of handle assembly 120. Inner shaft 142 is surrounded by outer shaft 144. Proximal end 146 of outer shaft 144 is operatively connected to knob 128 and lock 132. Inner shaft 142 is operatively connected to grip handle 126.

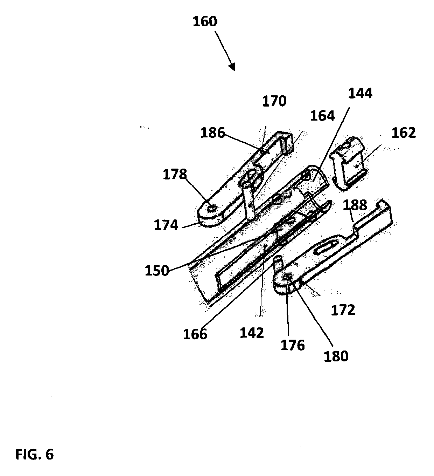

Rotatable end assembly 160 is connected to the distal end 148 of shaft assembly 140. As depicted in **FIG. 6****,** rotatable end assembly 160 includes arms 170/172, and deployment block 162. Outer shaft 144 includes openings (only one shown) 150. Pins 164/166 connect proximal portions 174/176 of arms 170/172 to shaft assembly 140. Arms 170/172 can include attachment holes 178/180 for connection to pins 164/166. Distal portions of arms 170/172 include inset portions 186/188. The inset portions 186/188 can approximate the arcuate shape of the bio-absorbable wound closure clip of the present disclosure. Deployment block 162 is located within the outer shaft 144 and operatively connected to inner shaft 142.

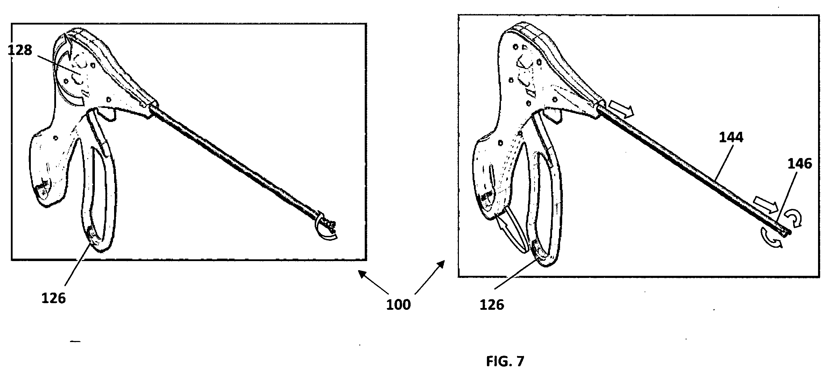

As shown in **FIG. 7**, upon contraction of grip handle 126, inner shaft 142 is moved toward proximal end 146 of outer shaft 144. Pins 164/166 move within openings (only one side shown) 150 drawing arms 170/172 toward each other and deployment block 162 toward the proximal end 146 of outer shaft 144. In some examples, the arms 170/172 do not touch but remain at a distance from each other even when fully closed, thereby preventing crushing of tissue. As further depicted in FIG. 7, inner shaft 142 and outer shaft 144 are rotatable at staggered angles by knob 128 around the horizontal axis of the surgical instrument 100 for accurate placement of a bio-absorbable wound closure clip.

In some examples, the surgical instrument of the present disclosure can be in the form of forceps having cupped distal ends specifically arced to hold the arcuate body of a bio-absorbable wound closure clip as described herein. In some examples, the distal ends of the forceps are configured for intentionally avoiding complete closure (e.g., the forceps distal ends do not close or touch together completely) thereby preventing or minimizing the likelihood of crushing of the tissue by the force of the forceps.

In use, the bio-absorbable wound closure clip of the present disclosure can be placed in the inset portions of the arms of the surgical instrument of the present disclosure prior to or at the time of deployment of the bio-absorbable wound closure clip, such that the arcuate body or portion of the bio-absorbable wound closure clip is held by the arcuate inset portions of the arms. The knob of the surgical instrument can then be used to accurately position the bio-absorbable wound closure clip and the grip handle of the surgical instrument can be pulled toward the stationary handle thereby pushing the deployment block forward and arms of the surgical instrument inward deforming the bio-absorbable wound closure clip while penetrating the tissue to bring the sides of a wound into proximal contact for healing.

In some examples at least a single bio-absorbable wound closure clip can be used to close a wound. In some examples, two or more bio-absorbable wound closure clips can be used to join tissues together or close a wound.

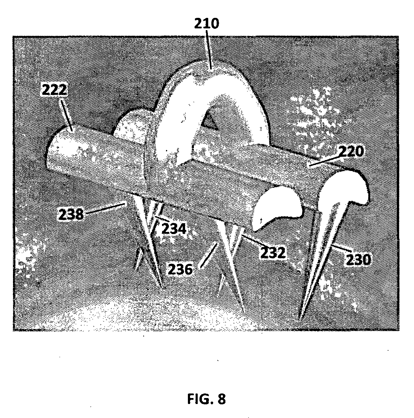

**FIG.** 8 depicts an embodiment of a bio-absorbable wound closure clip that can be deployed by the surgical instrument of the present disclosure. Bio-absorbable wound closure clip 200 includes an arcuate portion 210, horizontal arms 220/222, and prongs or teeth 230/232/234/236/238. As depicted in **FIG. 8**, arcuate portion 210 can operatively connect horizontal arms 220/222. The arcuate portion 201 can connect horizontal arms 220/222 at the middle of the horizontal arms 220/222. In embodiments, the arcuate portion 201 can connect the horizontal arms 220/222 at one end or the other of the horizontal arms 220/222. In embodiments the arcuate portion 201 can connect a first horizontal arm at one end, and the second horizontal arm at the opposite end.

Horizontal arms 220/222 include prongs or teeth 230/232/234/236/238 extending downward (opposite the location of the arcuate portion 210) therefrom. The prongs or teeth 230/232/234 on horizontal arm 220 are spaced one off from the prongs or teeth 236/238 of horizontal arm 222. Prongs or teeth 230/232/234/236/238 are angled downward from horizontal arms 220/222 and toward the midline of arcuate portion 210. In embodiments, the prongs or teeth 230/232/234/236/238 may be perpendicular to the horizontal arms 220/222. In some embodiments, the prongs or teeth 230/232/234/236/238 may be extend at the same angle from the horizontal arms 220/222. In some embodiments, the prongs or teeth 230/232/234/236/238 can extend at different angles from the horizontal arms 220/222. Although depicted with two and three prongs or teeth respectively, horizontal arms 220/222 can include any number of prongs or teeth extending therefrom.

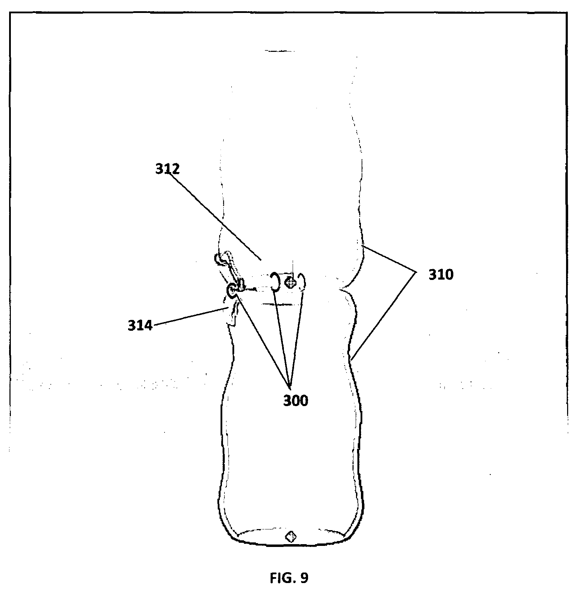

Tubular tissue structures within the body, for example, intestines, trachea, veins, and the like can be joined or closed using the clip(s) of the present disclosure. **FIG.** 9 depicts another example of a bio-absorbable wound closure clip. The bio-absorbable wound closure clip 300 can be deployed from the interior 312 of a tubular structure 310 or from the exterior 314 of the tubular structure 310. In examples, the bioabosrbable wound closure clip can be used to join a first portion of the tubular tissue structure and a second portion of the tubular tissue structure as depicted in **FIG. 9**. A plurality of bio-absorbable wound closure clips can be arranged/deployed/applied in a substantially circumferential (e.g., elliptical, circular, or ring-like) configuration or pattern joining the first and second portions of the tubular tissue structure. In examples, the bio-absorbable wound closure clips can be arranged in one or more additional or alternate configurations or patterns such as spirally, linearly, circularly and the like so as to join one or more portions of a tubular tissue structure. The bio-absorbable wound closure clip can be used to close fully or partially severed (through accident or surgically) tubular structures in the body. One or more bio-absorbable wound closure clips can be applied to the tubular structure. For instance, a plurality of bio-absorbable wound closure clips can be circumferentially deployed / disposed relative to each other about a first portion of a tubular structure (e.g., a first tubular structure) and a second portion of a tubular structure (e.g., the first tubular structure, or a second tubular structure) to secure the first portion (e.g., upper portion) of the tubular structure to the second portion (e.g., lower portion) of the tubular structure (e.g., such that the inner diameters of the plurality of bio-absorbable wound closure clips intersect a common spatial plane).

### Examples

The present technology is further illustrated by the following representative examples, which should not be construed as limiting in anyway.

### Example 1

### Degradation of bio-absorbable wound closure clip

20 bio-absorbable wound closure clips were formed from 99.5% magnesium (Sigma Aldrich, 13103). Ten of the bio-absorbable wound closure clips were coated with poly-ε-caprolactone (PCL) having an average molecular weight of 80,000 (Sigma Aldrich, 440744) as follows:
3% PCL w/v in methylene chloride
10 magnesium bio-absorbable wound closure clips immersed in PCL solution for 45 seconds followed by air drying at room temperature.

Immersion degradation was carried out in artificial saline as follows:
Artificial saline = 10:1 concentration of phosphate-buffered saline (PBS; Sigma Aldrich) with 0.92 grams/liter (g/l) xanthan gum;
Concentration of sodium chloride (NaCl) was maintained at 8 g/l

Magnesium and magnesium-PCL samples were suspended in separate solutions having an initial pH of 7.36.

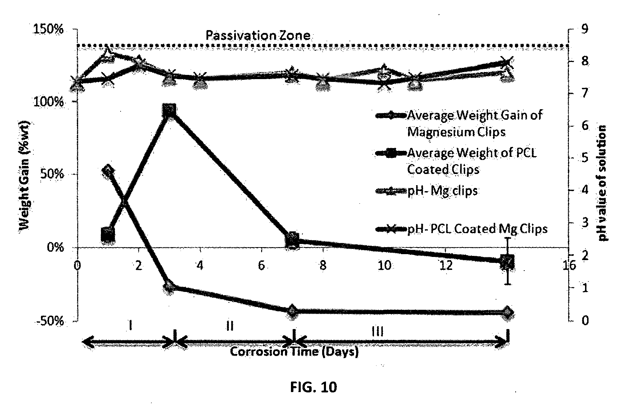

Immersion was carried out for two weeks followed by atmospheric drying for 48 hours. As depicted in **FIG. 10**.

### Example 2

### Wound Closure Clip Application and Strength

A longitudinal incision was made on one or both vocal cords of cadaveric pig larynges creating an epithelial flap. The bio-absorbable wound closure clips of the present disclosure were loaded onto the surgical instrument and inserted through a laryngoscope and oriented via an operating microscope. The surgical instrument was used to deploy and deform 3 to 5 bio-absorbable wound closure clips per larynx.

Assessment of the strength of the closure was determined based on vibration. The surgically secured cadaveric larynges were secured to a frame and the vocal folds were apposed to simulate vocal fold adduction during phonation. Air was pumped from below through the trachea to simulate subglottic air pressure and induce vibration of the vocal cords.

The ability of the bio-absorbable wound closure clip to hold securely with sustained traction or vibration was assessed. The bio-absorbable wound closure clips held securely for 15 minutes of vibration.

### Example 3

### In vivo Bio-Compatibility

Bio-absorbable wound closure clips were prepared in the form of clips. The clips ranged from 0.35 mm to 0.2 mm in thickness. Five types of clips were prepared as follows:
Type 1: Eight clips of unpolished 99.5% magnesium, 0.35 mm thick.
Type 2: Four clips polished 99.5% magnesium, 0.35 mm thick.
Type 3: Three clips polished 99.5% magnesium, 0.25 mm thick.
Type 4: Eight clips polished 99.5% magnesium, 0.2 mm.
Type 5: Four clips polished 99.5% magnesium coated with PCL as described above in Example 1, 0.2 mm thick.

Polished clips were polished with silicon carbide 2000 grit paper. Five pigs were anesthetized and positioned supine with the cervical spine slightly flexed. A laryngoscope was inserted transorally. Bio-absorbable wound closure clips were applied to pigs 1 to 5 as delineated in the chart below.

| **PIG** | **Closure Device Used** | | **Duration** | **Results post Sacrifice** | | **Remarks** |
|---|---|---|---|---|---|---|
| | **Ipsilateral** | **Contralateral** | | **Ipsilateral** | **Contralateral** | |
| **1** | 4 clips | 4 clips | 2 weeks | 1 clip | 0 clips | Type 1 |
| **2** | 4 clips, 1 non-absorbable suture | No incision | 2 weeks | 4 clips, 1 non-absorbable suture | NA | Type 2 |
| **3** | 3 clips, 1 absorbable suture | No incision | 3 weeks | 2 clips | NA | Type 3 |
| **4** | 3 clips | 5 clips | 3 weeks | 0 clips | 1 clip | Type 4 |
| **5** | 4 clips | Incision no closure device | 3 weeks | 1 clip | NA | Type 5 |

### Example 4

### Bio-Absorption

Rates of bio-absorption of clips of 99.5% magnesium *in situ* were tested using four pigs for three weeks. The results are shown in the table below.

| **Clip** | **No. Clips Inserted** | **No. Clips Present after 3 weeks** | **Attrition Rate, %** |
|---|---|---|---|
| **Unpolished Magnesium** | 12 | 5 | 58 |
| **Polished Magnesium** | 8 | 1 | 88 |
| **Gold Coated** | 3 | 0 | 100 |
| **PCL Coated** | 3 | 1 | 67 |

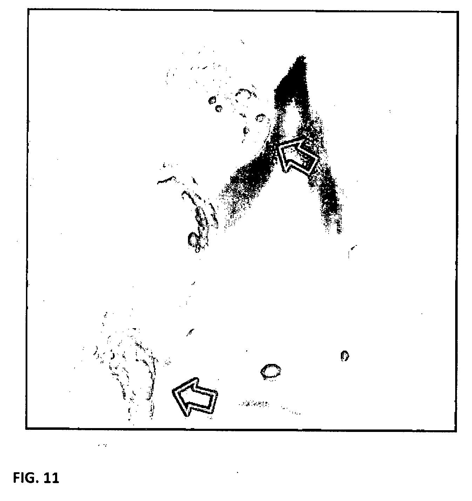

As shown in **FIG. 11**, the gold coated clips dissolved completely in a rapid amount of time, while the PCL coated clip remained.

### Example 5

### Effect of Inhalation

Five clips were implanted into the tracheae of two pigs beyond the endotracheal tube to mimic inhalation of the clips. After two weeks the larynx and entire lower airway were dissected but showed no sign of clips or inflammation.

### Example 6

### Deformation Under Load and Tensile Strength

To test the load bearing ability and tensile strength the clip was manually placed into silicone rubber sheets (simulating tissue) with surgical cusps forceps. Nylon threads were tied using a simple knot to the arcuate ends of the clip. The free ends of the nylon threads were looped around silicone sheets clamped on to the ends of a tensile tester. The tensile tester was set at a strain of 1 mm/s and readings were taken until load drops reached 40% of maximal value. A suture (n=4) was also tested for comparison purposes.

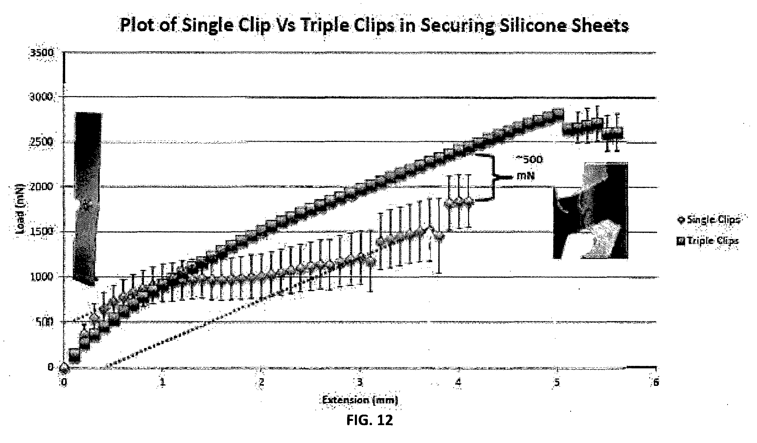

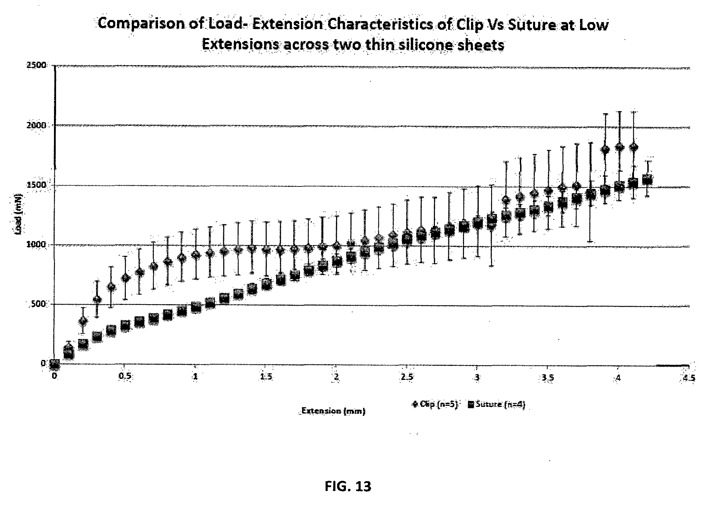

The results are graphically represented in FIGS. 12 and 13. As shown in **FIG. 12**, a single clip has a tensile strength of about 1800 mN. When three clips are used, the tensile holding strength of the set of clips is increased to about 2800 mN. As shown in **FIG. 13**, a clip has a greater tensile strength (about 1800 mN) as compared to a suture (about 1600 mN).

While various aspects and embodiments have been disclosed herein, other aspects and embodiments will be apparent to those skilled in the art. The various aspects and embodiments disclosed herein are for purposes of illustration and are not intended to be limiting, with the scope being indicated by the following claims.

## Claims

1. A wound closure clip comprising:
an arcuate body and a pair of arms (220, 222) operatively connected to said arcuate body (210) whereby said arcuate body is upstanding with respect to the longitudinal axis of said arms and wherein the arms (220, 222) are provided with a plurality of said teeth (230, 232, 234, 236, 238) which extend from said arms and are angled to project toward the midline of said arcuate body (210) **characterised in that** the wound closure clip is bio-absorbable and **in that** said teeth (230, 232, 234) on one horizontal arm (220) are spaced one off from the teeth (236, 238) of the other horizontal arm (222).

2. The bio-absorbable wound closure clip of claim 1, comprising a bio-absorbable material selected from the group comprising of bio-absorbable metals, bio-absorbable metal alloys, bio-absorbable polymers and bio-absorbable metal-polymer composites.

3. The bio-absorbable wound closure clip of claim 2, wherein said bio-absorbable material includes magnesium.

4. The bio-absorbable wound closure clip of claim 1, wherein said bio-absorbable wound closure clip is surface treated.

5. The bio-absorbable wound closure clip of claim 4, wherein said treatment is selected from at least one of polishing, bio-absorbable polymer coating, gold coating, hydrogen embrittlement, stress corrosion, acid corrosion, and abrasion.

6. The bio-absorbable wound closure clip of claim 1, wherein said bio-absorbable wound closure clip has a thickness between about 0.15 mm to about 0.4 mm.

7. The bio-absorbable wound closure clip of claim 1, wherein said bio-absorbable wound closure clip has a tensile strength between about 1500 mN to about 2500 mN.

8. The bio-absorbable wound closure clip of claim 1, wherein said bio-absorbable wound closure clip is made from a degradable material that degrades in situ in about 2 weeks.

9. A kit for wound closure comprising: a number of bio-absorbable wound closure clips according to claim 1.

## Patentansprüche

1. Wundverschlussclip, umfassend:
einen bogenförmigen Körper und
ein Paar Arme (220, 222), die in Wirkbeziehung mit dem bogenförmigen Körper (210) verbunden sind, wobei der bogenförmige Körper in Bezug auf die Längsachse der Arme aufrecht steht und wobei die Arme (220, 222) mit einer Mehrzahl der Zähne (230, 232, 234, 236, 238) versehen sind,
die sich von den Armen erstrecken und abgewinkelt sind, um zur Mittellinie des bogenförmigen Körpers (210) vorzuspringen, **dadurch gekennzeichnet, dass** der Wundverschlussclip bioabsorbierbar ist und dass die Zähne (230, 232, 234) an einem horizontalen Arm (220) um einen von den Zähnen (236, 238) des anderen horizontalen Arms (222) beabstandet sind.

2. Bioabsorbierbarer Wundverschlussclip nach Anspruch 1, umfassend ein bioabsorbierbares Material, ausgewählt aus der Gruppe umfassend bioabsorbierbare Metalle, bioabsorbierbare Metalllegierungen, bioabsorbierbare Polymere und bioabsorbierbare Metall-Polymer-Verbundstoffe.

3. Bioabsorbierbarer Wundverschlussclip nach Anspruch 2, wobei das bioabsorbierbare Material Magnesium beinhaltet.

4. Bioabsorbierbar Wundverschlussclip nach Anspruch 1, wobei der bioabsorbierbare Wundverschlussclip eine Oberflächenbehandlung aufweist.

5. Bioabsorbierbarer Wundverschlussclip nach Anspruch 4, wobei die Behandlung ausgewählt ist aus wenigstens einem von Polieren, Beschichten mit bioabsorbierbarem Polymer, Beschichten mit Gold, Wasserstoffversprödung, Spannungskorrosion, Säurekorrosion und Abschleifung.

6. Bioabsorbierbarer Wundverschlussclip nach Anspruch 1, wobei der bioabsorbierbare Wundverschlussclip eine Dicke zwischen etwa 0,15 mm und etwa 0,4 mm aufweist.

7. Bioabsorbierbarer Wundverschlussclip nach Anspruch 1, wobei der bioabsorbierbare Wundverschlussclip eine Zugfestigkeit zwischen etwa 1500 mN und etwa 2500 mN aufweist.

8. Bioabsorbierbarer Wundverschlussclip nach Anspruch 1, wobei der bioabsorbierbare Wundverschlussclip aus einem abbaubaren Material hergestellt ist, dass in situ innerhalb von etwa 2 Wochen abgebaut wird.

9. Kit zur Wundverschließung, umfassend: eine Anzahl von bioabsorbierbaren Wundverschlussclips nach Anspruch 1.

## Revendications

1. Pince de fermeture de plaie comprenant :
un corps arqué et
une paire de bras (220, 222) raccordés en fonctionnement audit corps arqué (210), moyennant quoi ledit corps arqué est levé par rapport à l'axe longitudinal desdits bras, et où les bras (220, 222) sont dotés d'une pluralité desdites dents (230, 232, 234, 236, 238) qui s'étendent depuis lesdits bras et qui sont inclinés pour faire saillie vers la ligne médiane dudit corps arqué (210) **caractérisé en ce que** la pince de fermeture de plaie est bio-absorbable et **en ce que** lesdites dents (230, 232, 234) situées sur un bras horizontal (220) sont espacées des dents (236, 238) de l'autre bras horizontal (222).

2. Pince bio-absorbable de fermeture de plaie selon la revendication 1, comprenant un matériau bio-absorbable choisi dans l'ensemble comprenant des métaux bio-absorbables, des alliages métalliques bio-absorbables, des polymères bio-absorbables et des composites de polymère bio-absorbables.

3. Pince bio-absorbable de fermeture de plaie selon la revendication 2, dans laquelle ledit matériau bio-absorbable contient du magnésium.

4. Pince bio-absorbable de fermeture de plaie selon la revendication 1, ladite pince bio-absorbable de fermeture de plaie étant traitée en surface.

5. Pince bio-absorbable de fermeture de plaie selon la revendication 4, ledit traitement étant choisi parmi au moins soit le polissage, soit une enduction de polymère bio-absorbable, la dorure, la fragilisation à l'hydrogène, la corrosion par contrainte, la corrosion acide et l'abrasion.

6. Pince bio-absorbable de fermeture de plaie selon la revendication 1, ladite pince bio-absorbable de fermeture de plaie ayant une épaisseur comprise entre 0,15mm et 0,4 mm environ.

7. Pince bio-absorbable de fermeture de plaie selon la revendication 1, ladite pince bio-absorbable de fermeture de plaie ayant une résistance à la traction comprise entre 1 500mN et 2 500 mN environ.

8. Pince bio-absorbable de fermeture de plaie selon la revendication 1, ladite pince bio-absorbable de fermeture de plaie étant constituée d'un matériau dégradable qui se dégrade in situ en près de 2 semaines.

9. Kit de fermeture de plaie contenant : un certain nombre de pinces bio-absorbables de fermeture de plaie selon la revendication 1.
